(19) Europäisches Patentamt
European Patent Office
Office européen des brevets

(11) Numéro de publication: **0 018 857**
**B1**

# (12) FASCICULE DE BREVET EUROPEEN

(45) Date de publication du fascicule du brevet: **19.01.83**

(21) Numéro de dépôt: **80400050.3**

(22) Date de dépôt: **15.01.80**

(51) Int. Cl.³: **A 61 K 31/495,** C 07 D 471/14 // (C07D471/14, 235/00, 221/00, 209/00),(C07D471/14, 239/00, 221/00, 209/00)

(54) **Pyrimido et imidazo-pyrido-indole-diones, leur préparation et leur application en thérapeutique.**

(30) Priorité: **26.04.79 FR 7910653**

(43) Date de publication de la demande: **12.11.80 Bulletin 80/23**

(45) Mention de la délivrance du brevet: **19.01.83 Bulletin 83/3**

(84) Etats contractants désignés: **AT BE CH DE FR GB IT LU NL SE**

(56) Documents cités:
**CITED DOCUMENTS (56):**
**JOURNAL OF ORGANIC CHEMISTRY, vol. 28, 1963 »Investigations in Heterocycles. XIV. 2- and 3-Azaoctahydroindolo (2,3alpha)quinolizines«, pages 3210—3212.**

**Le dossier contient des informations techniques présentées postérieurement au dépôt de la demande et ne figurant pas dans le présent fascicule.**

(73) Titulaire: **SYNTHELABO, 58 rue de la Glacière, F-75013 Paris (FR)**

(72) Inventeur: **Koletar Gabor, Istvan, 212, Dogwood Lane, Berkeley Heights, New Jersey 07922 (US)**
Inventeur: **Frost, Jonathan, 122, Résidence "Les Eaux Vives", F-91120 Palaiseau (FR)**
Inventeur: **Dupont, Régis, 141, Bd de la Gare, F-75013 Paris (FR)**
Inventeur: **Lardenois, Patrick, 18, rue Varengue, F-92340 Bourg La Reine (FR)**
Inventeur: **Morel, Claude, 15, rue de la Bergerie, F-91300 Massy (FR)**
Inventeur: **Najer, Henry, 2, avenue Emile Acollas, F-75007 Paris (FR)**

(74) Mandataire: **Thouret-Lemaitre, Elisabeth, Service Brevets - SYNTHELABO 58, rue de la Glacière, F-75621 Paris Cedex 13 (FR)**

**EP 0 018 857 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Pyrimido et imidazo-pyrido-indole-diones, leur preparation et leur application en therapeutique

La présente invention concerne des dérivés de pyrimido et imidazo-pyrido-indole-diones, leur préparation et leur application en thérapeutique.

Les composés de de l'invention, sous la forme de racémates ou d'isomères optiquement actifs, répondent à la formule

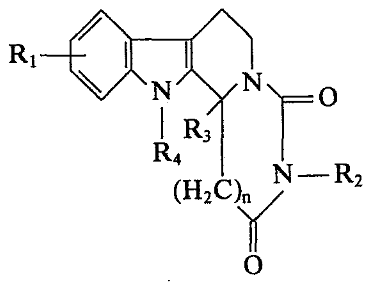

(I)

dans laquelle

n est 0 ou 1,
$R_1$ est un atome d'hydrogène ou d'halogène, un radical alkyle ou alcoxy ou le groupe $CF_3$,
$R_2$ est un atome d'hydrogène, un radical alkyle droit ou ramifié, un radical cycloalkylalkyle, un radical cycloalkyle, un radical dialkylaminoalkyle ou un radical phényle pouvant porter un atome d'halogène,
$R_3$ est un atome d'hydrogène ou un radical alkyle,
$R_4$ est un atome d'hydrogène, un radical alkyle ou un radical benzyle,

les alkyles et alcoxy ayant de 1 à 4 atomes de carbone, les cycloalkyles ayant de 3 à 6 atomes de carbone,
à l'exception des composés pour lesquels, simultanément,

a) n est 0,
$R_1$ est un atome d'hydrogène ou d'halogène ou un radical alkyle ou alcoxy,
$R_2$ est un atome d'hydrogène ou un radical alkyle, cycloalkyle ou cycloalkylalkyle,
$R_3$ est un atome d'hydrogène ou un radical alkyle et
$R_4$ est un atome d'hydrogène, ou bien
b) n est 1,
$R_1$ est un atome d'hydrogène ou d'halogène ou un radical alkyle ou alcoxy,
$R_2$ est un atome d'hydrogène ou un radical alkyle, cycloalkyle, cycloalkylalkyle ou phényle,
$R_3$ est un atome d'hydrogène ou un radical alkyle et
$R_4$ est un atome d'hydrogène.

Les composés ainsi exclus par les clauses a) et b) ci-dessus sont décrits dans la demande de brevet européen n° 8249.

Les composés préférés de l'invention sont ceux pour lesquels n = 0 ou 1, $R_1$ est H, Cl ou F, $R_2$ est un alkyle de 1 à 4 atomes de carbone, $R_3$ est H ou $CH_3$ et $R_4$ est H ou $CH_3$ et plus particulièrement ceux pour lesquels $R_1$ est Cl-9, $R_2$ est $CH_3$ ou $C_2H_5$, $R_3$ est H ou $CH_3$ et $R_4$ est H.

L'invention a également pour objets des médicaments contenant comme substances actives les composés définis ci-dessus, y compris ceux qui sont concernés par les clauses a) et b), mais à l'exception des quatre composés dans la formule desquels n = 1, $R_1$ = H, $R_2$ = H, $C_2H_5$, n-$C_4H_9$ ou $C_6H_5$, et $R_3$ et $R_4$ = H.

Ces derniers sont décrits par G. De Stevens et coll., dans J. Org. Chem., 28 (1963), 3210–12.

Selon l'invention, on prépare les composés (I) à partir des composés (II) de formule

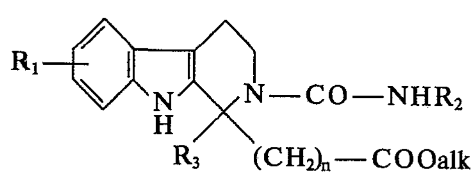

(II)

par cyclisation à l'aide d'acide chlorhydrique en milieu alcoolique à la température d'ébullition de l'alcool.

Les composés (I) dans lesquels $R_4$ est différent de H sont obtenus à partir des composés (I) dans

lesquels $R_4$ est H par alkylation ou benzylation directe de l'azote.

Par ailleurs les composés (I) dans lesquels $R_2$ est différent de H peuvent aussi être obtenus par alkylation directe de l'azote des composés (I) dans lesquels $R_2$ est H.

Les composés de formule (II) sont décrits dans la demande de brevet européen n° 21 857.

Les schémas de synthèse des composés (II) sont indiqués ci-après.

Schéma I

$R_3 \neq H$ n = 0 ou 1

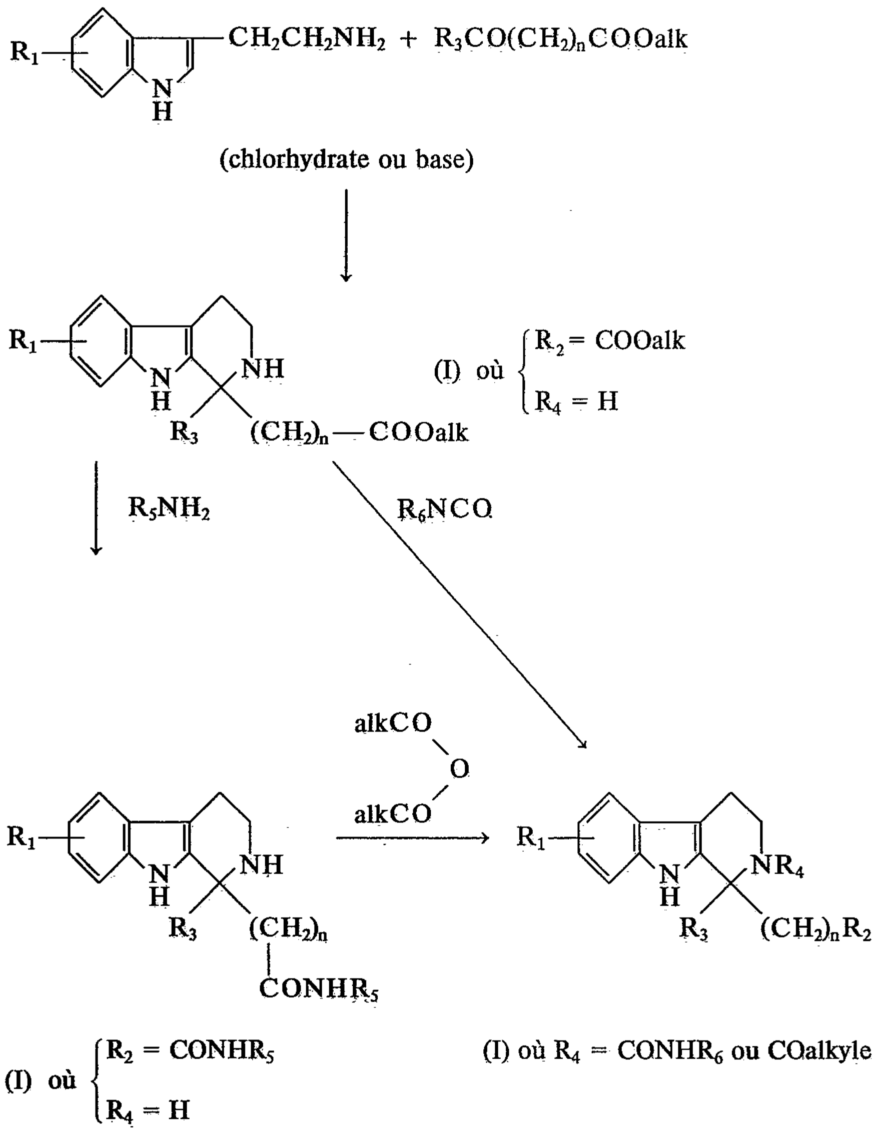

Schéma 2

$R_3 = H \quad n = 1$

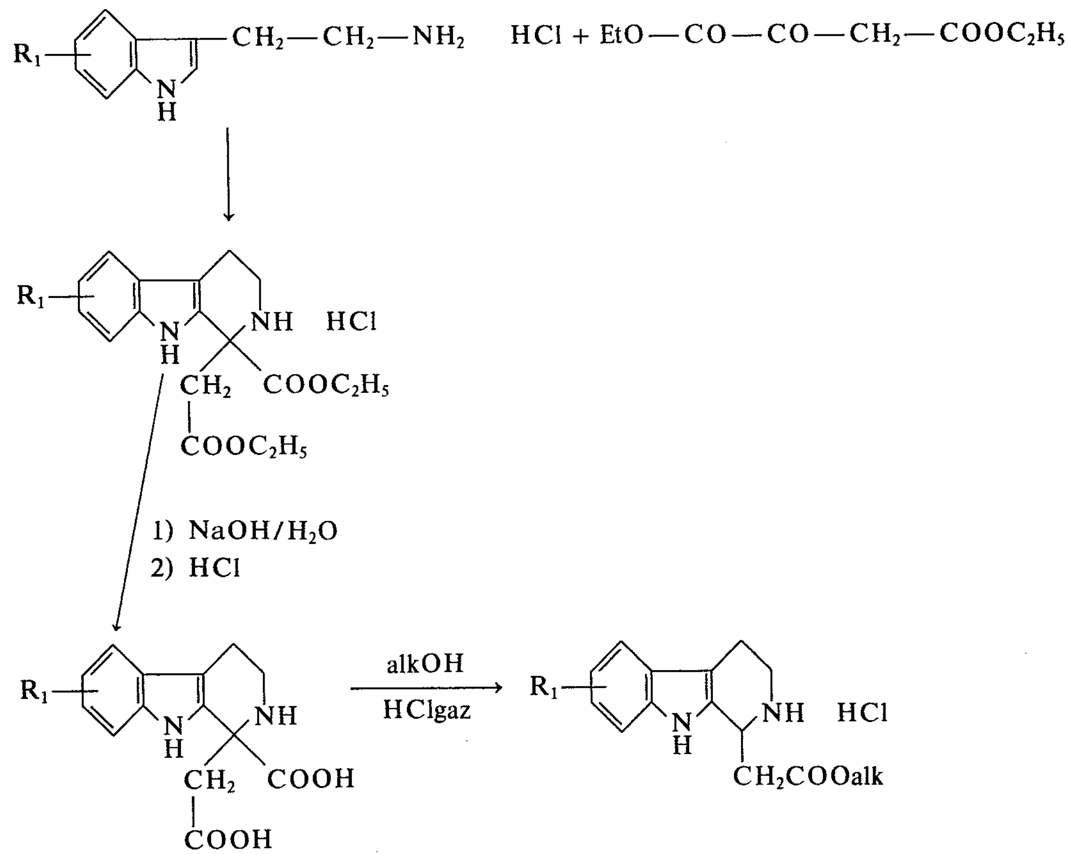


(I) où $\begin{cases} R_2 = COOalk \\ R_4 = H \end{cases}$

La transformation de l'ester (I) en amide (I) où $R_2 = CONHR_5$ est effectuée de la même manière que dans le schéma réactionnel 1; de même l'addition du radical $R_4 = CONHR_6$ sur les composés (I) où $R_2 = COOalk$ ou $CONHR_5$ est effectuée de la même manière que dans le schéma 1.

Enfin les composés (I) dans lesquels $R_1$ est $CF_3$ ou Br ont été préparés selon un procédé quelque peu différent (voir exemple 8), adapté à partir du procédé décrit pour $R_1 = H$ dans C.A. 60.5471 h.

Les composés pour lesquels $R_3 = H$ et $n = 0$ sont obtenus selon le mode de préparation décrit par Z. J. Vejdělek et coll., J. of Med. and Pharm. Chem., Vol. 3, N° 3 (1961) p. 427 – 440.

Les exemples suivants illustrent l'invention. Les microanalyses et les spectres IR et RMN confirment la structure des composés.

Exemple 1

Méthyl-2 hexahydro-2,3,5,6,11,11b imidazo[1',5':1,2]pyrido-[3,4-b]indole-dione-1,3

$[n = 0, R_1 = H, R_2 = CH_3, R_4 = H]$

1. Méthylaminocarbonyl-2 tétrahydro-2,3,4,9, 1H-pyrido[3,4-b]indole-1-carboxylate d'éthyle

Dans un ballon on place 48,9 g (0,2 mole) de tétrahydro-2,3,4,9 1H-pyrido[3,4-b]indole-1-carboxylate d'éthyle dans 900 ml de cyclohexane.

On agite et chauffe à la température de reflux sous atmosphère d'azote. On ajoute goutte à goutte, à ébullition en 15 mn une solution de 11,4 g (0,2 mole) d'isocyanate de méthyl dans 100 ml de cyclohexane. On porte à la température de reflux pendant 3 heures. On refroidit à la température ambiante. On essore et lave le solide avec du cyclohexane. On sèche sous vide. On obtient le composé qui fond à 158 – 162° C.

2. Méthyl-2 hexahydro-2,3,5,6,11,11b imidazo[1',5':1,2]pyrido [3,4-b]indole-dione-1,3

Dans un ballon on place 4,4 g (0,0146 mole) du composé obtenu précédemment et 400 ml d'éthanol. On dissout par chauffage. On ajoute 28 ml d'éthanol saturé par de l'acide chlorhydrique. On porte à la température de reflux pendant 1 heure 30. On laisse au repos pendant une nuit. On essore et lave le solide avec de l'éthanol glacé et recristallise le composé dans du nitrométhane en présence de noir de carbone. On isole le solide que l'on sèche sous vide à 110°C pendant 8 heures.

Le composé obtenu fond à 254–256°C.

Exemple 2

Chloro-9 méthyl-3 octahydro-1,2,3,4,6,7,12,12b-pyrimido[1',6':1,2]pyrido [3,4-b]indole-dione-2,4

[$n=1$, $R_1$=Cl-9, $R_2=CH_3$, $R_3$= H, $R_4$=H]

On place dans un ballon 10 g de chloro-6 tétrahydro-2,3,4,9 1H-pyrido[3,4-b]indole-1-acétate d'éthyle (préparé selon le brevet de la Demanderesse Pyrido-indoles déposé ce jour) et on ajoute 150 ml de cyclohexane et 2,5 ml (1,9 g) d'isocyanate de méthyle. On chauffe à la température de reflux pendant 1 heure. On refroidit dans un bain de glace. On essore. On obtient un solide blanc qui est repris par 150 ml d'éthanol. On agite et ajoute de l'éthanol saturé de gaz chlorhydrique (30 ml). On chauffe à reflux pendant 1 heure. On laisse refroidir. On essore. On recristallise dans de l'éthanol. F=304°C.

Exemple 3

Méthyl-3 octahydro-1,2,3,4,6,7,12,12b-pyrimido[1',6':1,2]pyrido [3,4-b]indole-dione-2,4

[$n=1$, $R_1$=H, $R_2=CH_3$, $R_3$=H, $R_4$=H]

1. Aminocarbonyl-2 tétrahydro-2,3,4,9 pyrimido[3,4-b]indole-1-acétate d'éthyle

On dissout 26 g (0,088 mole) de chlorhydrate de tétrahydro-2,3,4,9 1H pyrido[3,4-b]indole-1-acétate d'éthyle dans 1 l d'eau chaude. Le pH doit être de 4–5. Lorsque le milieu réactionnel est à 50°C, on ajoute une solution de 8,7 g (0,13 mole) de cyanate de sodium dans 150 cm³ d'eau. Le milieu réactionnel est maintenu à 50°C sous agitation. On agite 4 heures à 50°C et on laisse une nuit au repos à la température ambiante l'eau surnageante et sous-tirée. On ajoute 200 cm³ d'éther et agite, puis on sous-tire la solution éthérée, et recommence la même opération. Le produit attendu est très soluble dans l'éther. Les extraits éthérés sont réunis, lavés à l'eau acidulée afin d'éliminer les traces du produits de départ, puis à l'eau. On sèche sur $Na_2SO_4$ et évapore le solvant. On obtient une huile qui évolue en mousse par dessication plus poussée. Le produit est utilisé tel quel dans le stade suivant.

2. Octahydro-1,2,3,4,6,7,12,12b-pyrimido[1',6':1,2]pyrido [3,4-b]indole-dione-2,4

On dissout 11,1 g du composé précédent dans 250 cm³ d'éthanol à reflux. A cette température on ajoute une solution saturée de HCl gazeux dans 55 cm³ d'éthanol. On maintient le mélange à la température de reflux pendant 5 heures puis on laisse une nuit au repos à la température ambiante. Le produit est essoré, lavé avec EtOH puis à l'éther. On sèche. On obtient un produit très insoluble dans les solvants organiques. Après recristallisation dans du diméthylformamide, il fond à 365°C.

3. Méthyl-3 octahydro-1,2,3,4,6,7,12,12b-pyrimido[1',6':1,2] pyrido[3,4-b]indole-dione-2,4

A une solution de 8 g (0,031 mole) du composé précédent dans 300 cm³ de diméthylformamide, on ajoute 1,5 g (0,031 mole) d'hydrure de sodium en dispersion à 50% dans de l'huile. On agite pendant 1 heure 30 à l'abri de l'humidité puis on ajoute 4,5 g (0,031 mole) d'iodure de méthyle et on laisse le

milieu réactionnel une nuit à la température ambiante tout en agitant.

Le résidu minéral est éliminé par filtration puis le filtrat est évaporé. On reprend le résidu à l'eau, ce qui provoque sa concrétisation en solide blanc. Celui-ci est recristallisé dans de l'éthanol. Le composé pur, obtenu après recristallisation dans la méthyl-éthyl-cétone, fond à 246 – 248° C.

Tableau

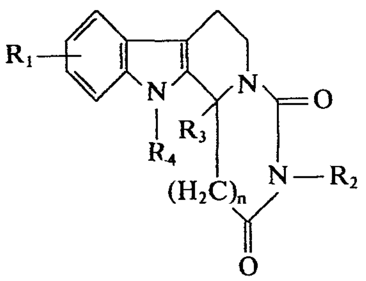

| Composé | $R_1$ | $R_2$ | $R_3$ | $R_4$ | n | F. (°C) |
|---|---|---|---|---|---|---|
| 1 | H | $CH_3$ | H | H | 1 | 246–248 |
| 2 | Cl-9 | $CH_3$ | H | H | 1 | 304 |
| 3 | H | $CH_3$ | H | $CH_3$ | 1 | 228–230 |
| 4 | H | $CH_3$ | $CH_3$ | H | 1 | 218 |
| 5 | Cl-9 | $CH_3$ | $CH_3$ | H | 1 | 265 |
| 6 | Cl-9 | $C_2H_5$ | H | H | 1 | 258 |
| 7 | H | n-$C_3H_7$ | H | H | 1 | 201–202 |
| 8 | H | $(CH_2)_2N(CH_3)_2$ | H | H | 1 | 260 (dec) |
| 9 | H | $CH_2$— 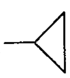 | H | H | 1 | 244 |
| 10 | H | i-$C_3H_7$ | H | H | 1 | 249 |
| 11 | H | H | H | H | 0 | 252–254 |
| 12 | H | $CH_3$ | H | H | 0 | 254–256 |
| 13 | H | $C_2H_5$ | H | H | 0 | 194–196 |
| 14 | H | n-$C_3H_7$ | H | H | 0 | 184–186 |
| 15 | H | i-$C_3H_7$ | H | H | 0 | 196–198 |
| 16 | H | n-$C_4H_9$ | H | H | 0 | 172–174 |
| 17 | H | t-$C_4H_9$ | H | H | 0 | 200–204 |
| 18 | H | $C_6H_5$ | H | H | 0 | 268–270 |
| 19 | H | p-Cl—$C_6H_4$ | H | H | 0 | 235–237 |
| 20 | H | $CH_3$ | $CH_3$ | H | 0 | 226 |
| 21 | F-9 | $CH_3$ | $CH_3$ | H | 1 | >280 |
| 22 | F-9 | $C_2H_5$ | $CH_3$ | H | 1 | 240 |
| 23 | F-9 | $CH_3$ | H | H | 1 | 249–250 |
| 24 | Cl-9 | $CH_3$ | H | $CH_3$ | 1 | 266–268 |
| 25 | H | $CH_3$ | H | $CH_2Ph$ | 1 | 198–199 |
| 27 | $CH_3$-9 | $C_2H_5$ | $CH_3$ | H | 1 | 215 |

Fortsetzung

| Composé | $R_1$ | $R_2$ | $R_3$ | $R_4$ | n | F. (°C) |
|---|---|---|---|---|---|---|
| 28 | $CH_3$-9 | $CH_3$ | $CH_3$ | H | 1 | 262 |
| 29 | $CH_3O$-9 | $CH_3$ | $CH_3$ | H | 1 | 252 |
| 32 | $CH_3O$-9 | $CH_3$ | H | H | 1 | 267–268 |
| 33 | $CH_3$-9 | $CH_3$ | H | H | 1 | 217 |
| 34 | $CF_3$-9 | $CH_3$ | H | H | 1 | 277 |
| 35 | Br-9 | $CH_3$ | H | H | 1 | 300 |

Les composés de l'invention ont été soumis à divers essais pharmacologiques.

Les composés ont en effet été soumis au test de l'anoxie hypobare chez la souris et au test de l'action sur la durée du sommeil induit par le 4-hydroxy-butyrate de sodium chez le rat curarisé.

Anoxie hypobare

Des souris de souche CDI sont maintenues dans une atmosphère appauvrie en oxygène, par réalisation d'un vide partiel (190 mm de mercure correspondant à 5,25% d'oxygène).

Le temps de survie des animaux est noté. Ce temps est augmenté par les agents capables de favoriser l'oxygénation tissulaire et en particulier cérébrale. Les composés étudiés sont administrés à plusieurs doses, par voie intrapéritonéale, 10 minutes avant l'essai. Les pourcentages d'augmentation du temps de survie par rapport aux valeurs obtenues chez les animaux témoins sont calculés. La dose moyenne (DAM), dose qui augmente le temps de survie de 100% est déterminée graphiquement.

La DAM des composés de l'invention varie de 0,5 à 20 mg/kg par voie i.p.

Action sur la duree du »sommeil«

Cette action a été déterminée par l'influence des composés sur la durée du »sommeil« induit par le 4-hydroxy-butyrate de sodium (GBH) chez la rat curarisé sous respiration artificielle dans lequel l'activité électrocorticographique est enregistrée par des électrodes corticales.

Les composés de l'invention diminuent la durée totale du sommeil de 15 à 35%.

L'étude pharmacologique des composés de l'invention montre qu'ils sont actifs dans l'épreuve d'anoxie hypobare chez la souris tout en n'étant que peu toxiques et qu'ils exercent une action significative éveillante dans le test du »sommeil« induit par le 4-hydroxy-butyrate de sodium.

Les composés de l'invention, possédant à la fois une activité antianoxique et une activité psychotrope, peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens, et le traitement des états dépressifs.

L'invention comprend par conséquent, toutes compositions pharmaceutiques, renfermant les composés et/ou leurs sels comme principes actifs, en association avec tous excipients appropriés à leur administration, en particulier par voie orale ou parentérale.

La posologie quotidienne peut aller de 10 à 1000 mg.

**Revendications pour les Etats contractants: BE, CH, DE, FR, GB, IT, LU, NL, SE**

1. Médicament caractérisé en ce qu'il contient comme substance activé un dérivé de pyrimido- ou imidazo-pyrido-indole, sous forme de racémate ou d'isomères optiquement actifs, répondant à la formule (I)

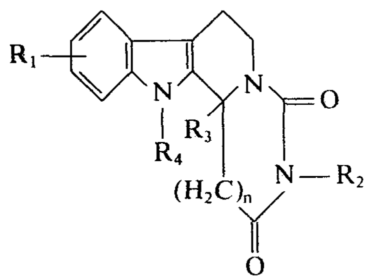

dans laquelle

n est 0 ou 1,
$R_1$ est un atome d'hydrogène ou d'halogène, un radical alkyle ou alcoxy ou le groupe $CF_3$,
$R_2$ est un atome d'hydrogène, un radical alkyle droit ou ramifié, un radical cycloalkylakyle, un radical cycloalkyle, un radical diakylaminoalkyle ou un radical phényle pouvant porter un atome d'halogène,
$R_3$ est un atome d'hydrogène ou un radical alkyle,
$R_4$ est un atome d'hydrogène, un radical alkyle ou un radical benzyle,

les alkyles et alcoxy ayant de 1 à 4 atomes de carbone, les cycloalkyles ayant de 3 à 6 atomes de carbone,
à l'exception des composés pour lesquels $n = 1$, $R_1 = H$, $R_2 = H$, $C_2H_5$, $nC_4H_9$ ou $C_6H_5$, $R_3$ et $R_4 = H$.

2. Médicament selon la revendication 1, caractérisé en ce qu'il contient un dérivé tel que défini dans la revendication 1 et dans la formule duquel n est 0 ou 1, $R_1$ est un atome d'hydrogène, de chlore ou de fluor, $R_2$ est un radical alkyle de 1 à 4 atomes de carbone, $R_3$ est un atome d'hydrogène ou un groupe méthyle et $R_4$ est un atome d'hydrogène ou un groupe méthyle.

3. Médicament selon la revendication 2, caractérisé en ce qu'il contient un dérivé tel que défini dans la revendication 1 et dans la formule duquel $R_1$ est un atome de chlore en postion 9, $R_2$ est un groupe méthyle ou éthyle, $R_3$ est un atome d'hydrogène ou un groupe méthyle et $R_4$ est un atome d'hydrogène.

4. Médicament caractérisé en ce qu'il contient comme substance active, sous forme de racémate ou d'isomères optiquement actifs, la chloro-9 méthyl-3 octahydro-1,2,3,4,6,7,12,12b-pyrimido[1,6':1,2]pyrido[3,4-b]indoledione-2,4.

5. Dérivés de pyrimido et imidazo-pyrido-indoles, sous la forme de racémates ou d'isomères optiquement actifs, répondant à la formule (I)

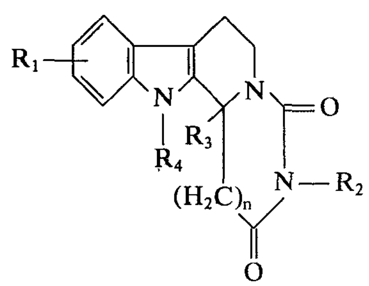

dans laquelle

n est 0 ou 1,
$R_1$ est un atome d'hydrogène ou d'halogène, un radical alkyle ou alcoxy ou le groupe $CF_3$,
$R_2$ est un atome d'hydrogène, un radical alkyle droit ou ramifié, un radical cycloalkylalkyle, un radical cycloalkyle, un radical diakylaminoakyle ou un radical phényle pouvant proter un atome d'halogène,
$R_3$ est un atome d'hydrogène ou un radical alkyle,
$R_4$ est un atome d'hydrogène, un radical alkyle ou un radical benzyle,

les alkyles et alcoxy ayant de 1 à 4 atomes de carbone, les cycloalkyles ayant de 3 à 6 atomes de carbone,
à l'exception des dérivés dans la formule desquels, simultanément,

a) n est 0,
$R_1$ est un atome d'hydrogène ou d'halogène ou un radical alkyle ou alcoxy,
$R_2$ est un atome d'hydrogène ou un radical alkyle, cycloalkyle ou cycloalkylalkyle,

$R_3$ est un atome d'hydrogène ou un radical alkyle et
$R_4$ est un atome d'hydrogène, ou bien

b) n est 1,
$R_1$ est un atome d'hydrogène ou d'halogène ou un radical alkyle ou alcoxy,
$R_2$ est un atome d'hydrogène ou un radical alkyle, cycloalkyle, cycloalkylalkyle ou phényle,
$R_3$ est un atome d'hydrogène ou un radical alkyle et
$R_4$ est un atome d'hydrogène.

6. Dérivés selon la revendication 5, dans la formule desquels n est 0 ou 1, $R_1$ est un atome d'hydrogène, de chlore ou de fluor, $R_2$ est un radical alkyle de 1 à 4 atomes de carbone, $R_3$ est un atome d'hydrogène ou un groupe méthyle et $R_4$ est un groupe méthyle.

7. Procédé de préparation des dérivés selon la revendication 5, caractérisé en ce qu'on cyclise un composé de formule (II)

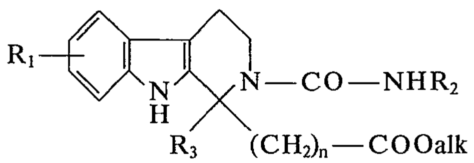

n, $R_1$, $R_2$, $R_3$ et $R_4$ étant tels que définis dans la revendication 5, et alk étant un radical alkyle inférieur, à l'aide d'acide chlorhydrique en milieu alcoolique à la température d'ébullition de l'alcool, puis si on le désire on alkyle ou benzyle le composé (I) obtenu dans lequel $R_4$ est H.

**Revendication pour l'Etat contractant: AT**

Procédé de préparation de dérivés de pyrimido et imidazo-pyrido-indoles, sous la forme de racémates ou d'isomères optiquement actifs, répondant à la formule (I).

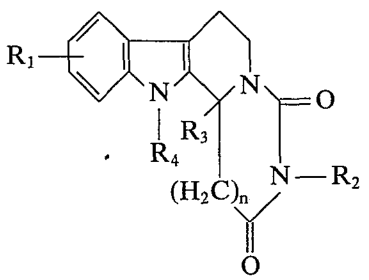

dans laquelle

n est 0 ou 1,
$R_1$ est un atome d'hydrogène ou d'halogène, un radical alkyle ou alcoxy ou le groupe $CF_3$,
$R_2$ est un atome d'hydrogène, un radical alkyle droit ou ramifié, un radical cycloalkylalkyle, un radical cycloalkyle, un radical dialkylaminoalkyle ou un radical phényle pouvant porter un atome d'halogène,
$R_3$ est un atome d'hydrogène ou un radical alkyle,
$R_4$ est un atome d'hydrogène, un radical alkyle ou un radical benzyle,

les alkyles et alcoxy ayant de 1 à 4 atomes de carbone, les cycloalkyles ayant de 3 à 6 atomes de carbone, à l'exception des dérivés dans la formule desquels, simultanément,

a) n est 0,
$R_1$ est un atome d'hydrogène ou d'halogène ou un radical alkyle ou alcoxy,
$R_2$ est un atome d'hydrogène ou un radical alkyle, cycloalkyle ou cycloalkylalkyle,
$R_3$ est un atome d'hydrogène ou un radical alkyle et
$R_4$ est un atome d'hydrogène, ou bien

b) b est 1,
$R_1$ est un atome d'hydrogène ou d'halogène ou un radical alkyle ou alcoxy,
$R_2$ est un atome d'hydrogène ou un radical alkyle, cycloalkyle, cycloalkylalkyle ou phényle,
$R_3$ est un atome d'hydrogène ou un radical alkyle et
$R_4$ est un atome d'hydrogène,

procédé caractérisé en ce qu'on cyclise un composé de formule (II)

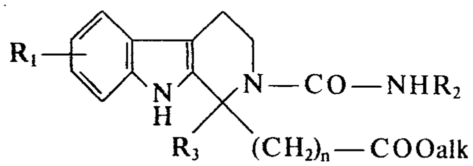

a l'aide d'acide chlorhydrique en milieu alcoolique à la température d'ébullition de l'alcool, puis si on le désire on alkyle ou benzyle le composé (I) obtenu dans lequel $R_4$ est H, n, $R_1$, $R_2$, $R_3$ et $R_4$ étant tels que définis ci-dessus et alk étant un radical alkyle inférieur.

**Patentansprüche für die Vertragsstaaten: BE, CH, DE, FR, GB, IT, LU, NL, SE**

1. Heilmittel, dadurch gekennzeichnet, daß es als Wirkstoff ein Pyrimido- oder Imidazo-Pyridoindolderivat in Form des Racemats oder der optisch aktiven Isomeren der allgemeinen Formel

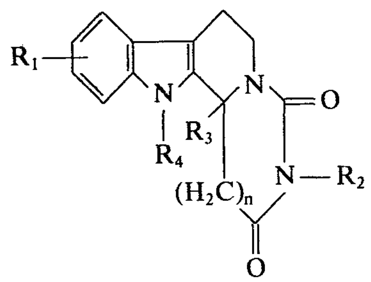

(I)

enthält, in welcher

n für 0 oder 1,
$R_1$ für Wasserstoff, Halogen, einen Alkyl- oder Alkoxyrest oder für die Gruppe $CF_3$,
$R_2$ für Wasserstoff, einen geradkettigen oder verzweigten Alkylrest, einen Cycloalkylalkyl-, Cycloalkyl-, Dialkylaminoalkyl- oder einen gegebenenfalls durch ein Haolgen substituierten Phenylrest,
$R_3$ für Wasserstoff oder einen Alkylrest und
$R_4$ für Wasserstoff, einen Alkyl- oder Benzylrest steht,

wobei die Alkyl- und Alkoxygruppen 1 bis 4 C-Atome und die Cycloalkylgruppen 3 bis 6 C-Atome enthalten,
mit der Ausnahme von Derivaten der obigen Formel, bei welchen $n = 1$, $R_1 = H$, $R_2 = H$, $C_2H_5$, $n\text{-}C_4H_9$ oder $C_6H_5$, $R_3$ und $R_4 = H$.

2. Heilmittel nach Anspruch 1, dadurch gekennzeichnet, daß es eine Verbindung der allgemeinen Formel I enthält, in welcher n für 0 oder 1, $R_1$ für Wasserstoff, Chlor oder Fluor, $R_2$ für einen Alkylrest mit 1 bis 4 C-Atomen, $R_3$ für Wasserstoff oder eine Methylgruppe und $R_4$ für Wasserstoff oder eine Methylgruppe steht.

3. Heilmittel nach Anspruch 2, dadurch gekennzeichnet, daß es eine Verbindung der allgemeinen Formel I enthält, in welcher $R_1$ für Chlor in 9-Stellung, $R_2$ für eine Methyl- oder Ethylgruppe, $R_3$ für Wasserstoff oder eine Methylgruppe und $R_4$ für Wasserstoff steht.

4. Heilmittel, dadurch gekennzeichnet, daß es als Wirkstoff in Form des Racemats oder der optisch aktiven Isomeren das 9-Chlor-3-methyl-1,2,3,4,6,7,12,12b-octahydro-[1,6':1,2]-pyrimido-[3,4-b]-pyrido-2,4-indoldion enthält.

5. Pyrimido- und Imidazo-Pyridoindolderivate in Form ihrer Racemate oder optisch aktiven Isomeren, die der allgemeinen Formel

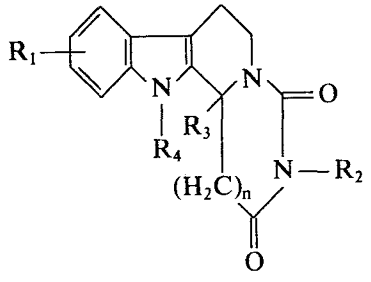

(I)

entsprechen, in welcher

n für 0 oder 1,
$R_1$ für Wasserstoff, Halogen, einen Alkyl- oder Alkoxyrest oder für die Gruppe $CF_3$,
$R_2$ für Wasserstoff, einen geradkettigen oder verzweigten Alkylrest, einen Cycloalkylalkyl-, Cycloalkyl-, Dialkylaminoalkyl- oder einen gegebenenfalls durch ein Halogen substituierten Phenylrest,
$R_3$ für Wasserstoff oder einen Alkylrest und
$R_4$ für Wasserstoff, einen Alkyl- oder Benzylrest steht,

wobei die Alkyl- und Alkoxygruppen 1 bis 4 C-Atome und die Cycloalkylgruppen 3 bis 6 C-Atome enthalten,
mit der Ausnahme von Derivaten der obigen Formel, bei welchen gleichzeitig

a) n für 0 steht und
$R_1$ Wasserstoff, Halogen, einen Alkyl- oder Alkoxyrest,
$R_2$ Wasserstoff, einen Alkyl-, Cycloalkyl- oder Cycloalkylalkylrest,
$R_3$ Wasserstoff oder einen Alkylrest und
$R_4$ Wasserstoff bedeutet, oder
b) n für 1 steht und
$R_1$ Wasserstoff, Halogen oder einen Alkyl- oder Alkoxyrest,
$R_2$ Wasserstoff oder einen Alkyl-, Cycloalkyl-, Cycloalkylalkyl- oder Phenylrest,
$R_3$ Wasserstoff oder einen Alkylrest und
$R_4$ Wasserstoff bedeutet.

6. Derivate nach Anspruch 5, die der Formel I entsprechen, in welcher n für 0 oder 1, $R_1$ für Wasserstoff, Chlor oder Fluor, $R_2$ für einen Alkylrest mit 1 bis 4 C-Atomen, $R_3$ für Wasserstoff oder die Methylgruppe und $R_4$ für die Methylgruppe steht.

7. Verfahren zur Herstellung von Derivaten nach Anspruch 5, dadurch gekennzeichnet, daß man eine Verbindung der allgemeinen Formel

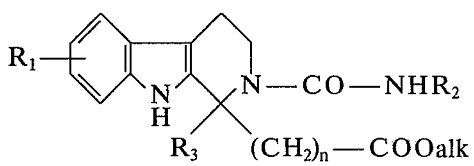

(II)

in welcher n, $R_1$, $R_2$ und $R_3$ die in Anspruch 5 angegebene Bedeutung haben und Alk für einen niederen Alkylrest steht, mit Hilfe von Chlorwasserstoffsäure in alkoholischem Milieu bei der Siedetemperatur des Alkohols cyclisiert und anschließend gegebenenfalls die erhaltenen Verbindungen der Formel I, in welchen $R_4$ für H steht, alkyliert oder benzyliert.

**Patentanspruch für den Vertragsstaat: AT**

Verfahren zur Herstellung von Pyrimido- und Imidazo-Pyridoindoldionen in Form ihrer Racemate oder optisch aktiven Isomeren der allgemeinen Formel

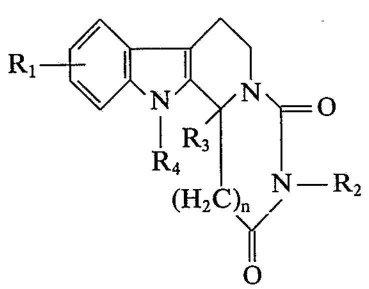

(I)

in welcher

n für 0 oder 1,
$R_1$ für Wasserstoff, Halogen, einen Alkyl- oder Alkoxyrest oder für die Gruppe $CF_3$,
$R_2$ für Wasserstoff, einen geradkettigen oder verzweigten Alkylrest, einen Cycloalkylalkyl-, Cycloalkyl-, Dialkylaminoalkyl- oder einen gegebenenfalls durch ein Halogen substituierten Phenylrest,

$R_3$ für Wasserstoff oder einen Alkylrest und
$R_4$ für Wasserstoff, einen Alkyl- oder Benzylrest steht,

wobei die Alkyl- und Alkoxygruppen 1 bis 4 C-Atome und die Cycloalkylgruppen 3 bis 6 C-Atome enthalten,
mit der Ausnahme von Derivaten der obigen Formel, bei welchen gleichzeitig

(a) n für 0 steht und
$R_1$ Wasserstoff, Halogen, einen Alkyl- oder Alkoxyrest,
$R_2$ Wasserstoff, einen Alkyl-, Cycloalkyl- oder Cycloalkylalkylrest,
$R_3$ Wasserstoff oder einen Alkylrest und
$R_4$ Wasserstoff bedeutet oder
b) n für 1 steht und
$R_1$ Wasserstoff, Halogen oder einen Alkyl- oder Alkoxyrest,
$R_2$ Wasserstoff oder einen Alkyl-, Cycloalkyl-, Cycloalkylalkyl- oder Phenylrest,
$R_3$ Wasserstoff oder einen Alkylrest und
$R_4$ Wasserstoff bedeutet,

dadurch gekennzeichnet, daß man eine Verbindung der allgemeinen Formel

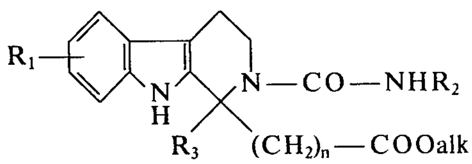
(II)

in welcher n, $R_1$, $R_2$ und $R_3$ die obengenannte Bedeutung haben und Alk für einen niederen Alkylrest steht, mit Hilfe von Chlorwasserstoffsäure in alkoholischem Milieu bei der Siedetemperatur des Alkohols cyclisiert und gegebenenfalls die erhaltenen Verbindungen der Formel I, in welchen $R_4$ für H steht, alkyliert oder benzyliert.

**Claims for the contracting states: BE, CH, DE, FR, GB, IT, LU, NL, SE**

1. A drug characterized in that it contains, as an active substance, a derivative of a pyrimido or imidazo-pyrido-indole, in form of racemate or optically active isomers, corresponding to formula (I)

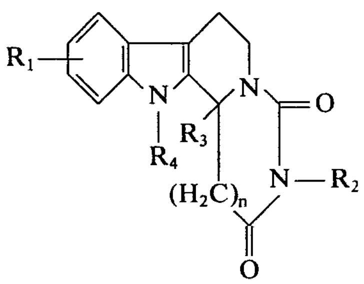

wherein

n is 0 or 1,
$R_1$ is a hydrogen or halogen atom, an alkyl or alkoxy radical or the $CF_3$ group,
$R_2$ is a hydrogen atom, a straight or branched alkyl radical, a cycloalkylalkyl radical, a cycloalkyl radical, a dialkylaminoalkyl radical or a phenyl radical which may bear a halogen atom,
$R_3$ is a hydrogen atom or an alkyl radical,
$R_4$ is a hydrogen atom or an alkyl or benzyl radical,

said alkyls and alkoxy having 1 to 4 carbon atoms, said cycloalkyls having 3 to 6 carbon atoms, with the exception of the compounds for which $n = 1$, $R_1 = H$, $R_2 = H$, $C_2H_5$, n $C_4H_9$ or $C_6H_5$, $R_3$ and $R_4 = H$.

2. A drug according to claim 1, characterized in that it contains a derivative as specified in claim 1 and in the formula of which n is 0 or 1, $R_1$ is a hydrogen, chlorine or fluorine atom, $R_2$ is an alkyl radical having 1 to 4 carbon atoms, $R_3$ is a hydrogen atom or a methyl group and $R_4$ is a hydrogen atom or a methyl group.

3. A drug according to claim 2, characterized in that it contains a derivative as specified in claim 1 and in the formula of which $R_1$ is a chlorine atom at position 9, $R_2$ is a methyl or ethyl group, $R_3$ is a hydrogen atom or a methyl group and $R_4$ is a hydrogen atom.

4. A drug characterized in that it contains as an active substance, in forme of recemate or optically active isomers, the 9-chloro-3-methyl-1,2,3,4,6,7,12,12b-octahydro-pyrimido[1,6':1,2]pyrido[3,4-b]indole-2,4-dione.

5. Pyrimido and imidazo-pyrido-indole derivatives, in form of racemates or optically active isomers, corresponding to formula (I)

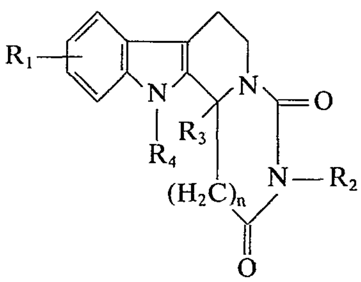

wherein

n is 0 or 1,
$R_1$ is a hydrogen or halogen atom, an alkyl or alkoxy radical, or the $CF_3$ group,
$R_2$ is a hydrogen atom, a straight or branched alkyl radical, a cycloalkylalkyl radical, a cycloalkyl radical, a dialkylaminoalkyl radical or a phenyl radical which may bear a halogen atom,
$R_3$ is a hydrogen atom or an alkyl radical,
$R_4$ is a hydrogen atom, an alkyl radical or a benzyl radical,

said alkyls and alkoxy having 1 to 4 carbon atoms, said cycloalkyl having 3 to 6 carbon atoms, with the exception of the compounds in the formula of which, simulataneously, either

a) n is 0,
$R_1$ is a hydrogen or halogen atom or an alkyl or alkoxy radical,
$R_2$ is a hydrogen atom or an alkyl, cycloalkyl or cycloalkylalkyl radical,
$R_3$ is a hydrogen atom or an alkyl radical and
$R_4$ is a hydrogen atom, or

b) n is 1,
$R_1$ is a hydrogen or halogen atom or an alkyl or alkoxy radical,
$R_2$ is a hydrogen atom or an alkyl, cycloalkyl, cycloalkylalkyl or phenyl radical,
$R_3$ is a hydrogen atom or an alkyl radical and,
$R_4$ is a hydrogen atom.

6. Derivatives according to claim 5, in the formula of which n is 0 or 1, $R_1$ is a hydrogen, chlorine or fluorine atom, $R_2$ is an alkyl radical having 1 to 4 carbon atoms, $R_3$ is a hydrogen atom or a methyl group and $R_4$ is a methyl group.

7. A process for preparing derivatives according to claim 5, characterized in that a compound of formula (II)

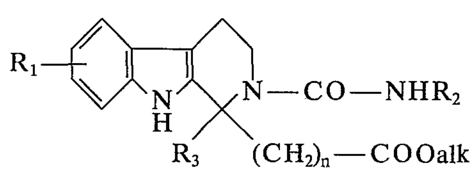

wherein n, $R_1$, $R_2$, $R_3$ and $R_4$ are as specified in claim 5 and alk is a lower alkyl radical, is cyclized with hydrochloric acid in alcoholic medium at the boiling temperature of the alcohol, and then, if desired, the compound thus obtained (I), wherein $R_4$ is H, is alkylated or benzylated.

**Claim for the contracting state: AT**

A process for preparing pyrimido and imidazo-pyrido-indole derivatives, in form of racemates or optically active isomers, corresponding to formula (I)

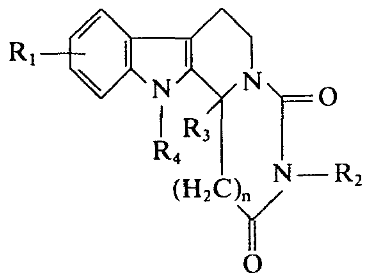

wherein

n is 0 or 1,
$R_1$ is a hydrogen or halogen atom, an alkyl or alkoxy radical, or the $CF_3$ group,
$R_2$ is a hydrogen atom, a straight or branched alkyl radical, a cycloalkylalkyl radical, a cycloalkyl radical, a dialkylaminoalkyl radical or a phenyl radical which may bear a halogen atom,
$R_3$ is a hydrogen atom or an alkyl radical,
$R_4$ is a hydrogen atom, an alkyl radical or a benzyl radical,

said alkyls and alkoxy having 1 to 4 carbon atoms, said cycloalkyls having 3 to 6 carbon atoms, with the exception of the compounds in the formula of which, simultaneously, either

a) n is 0,
$R_1$ is a hydrogen or halogen atom or an alkyl or alkoxy radical,
$R_2$ is a hydrogen atom or an alkyl, cycloalkyl or cycloalkylalkyl radical,
$R_3$ is a hydrogen atom or an alkyl radical and
$R_4$ is a hydrogen atom, or
b) n is 1,
$R_1$ is a hydrogen or halogen atom or an alkyl or alkoxy radical,
$R_2$ is a hydrogen atom or an alkyl, cycloalkyl, cycloalkylalkyl or phenyl radical,
$R_3$ is a hydrogen atom or an alkyl radical, and
$R_4$ is a hydrogen atom,

characterized in that a compound of formula (II)

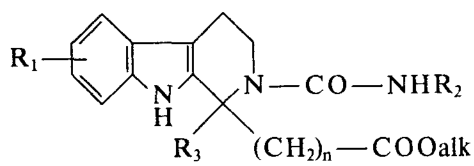

is cyclized with hydrochloric acid in alcoholic medium at the boiling temperature of the alcohol, and then, if desired, the compound thus obtained (I), wherein $R_4$ is H, is alkylated or benzylated, n, $R_1$, $R_2$, $R_3$ and $R_4$ being as specified above and alk being a lower alkyl radical.